(19) Europäisches Patentamt / European Patent Office / Office européen des brevets

(11) **EP 2 628 790 B1**

(12) **EUROPEAN PATENT SPECIFICATION**

(45) Date of publication and mention
of the grant of the patent:
**20.01.2016 Bulletin 2016/03**

(51) Int Cl.:
**B01L 3/00** (2006.01)    **C12M 3/06** (2006.01)

(21) Application number: **12000996.4**

(22) Date of filing: **16.02.2012**

(54) **Micro fluidic system for simulating in vivo-equivalent cell barriers**

Mikrofluidisches System zur Simulation in-vivo-identischer Zellbarrieren

Système micro-fluidique pour simuler des barrières cellulaires in vivo-équivalentes

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.08.2013 Bulletin 2013/34**

(73) Proprietor: **Hochschule Kaiserslautern**
**67657 Kaiserslautern (DE)**

(72) Inventors:
• **Schäfer, Karl-Herbert, Prof. Dr. med. habil.,**
**66459 Kirkel (DE)**
• **Saumer, Monika, Prof. rer. nat.,**
**76351 Linkenheim-Hochstetten (DE)**
• **Gress, Carola, Dipl.-Ing. (FH)**
**66399 Ommersheim (DE)**
• **Jeziorski, Markus, Dipl.-Ing. (FH)**
**40764 Langenfeld (DE)**

(74) Representative: **Patentanwälte Dr. Keller,**
**Schwertfeger**
**Westring 17**
**76829 Landau (DE)**

(56) References cited:
**WO-A2-2009/102751    US-A- 5 750 329**

• **GLAUM R ET AL: "Tissue engineering of composite grafts: Cocultivation of human oral keratinocytes and human osteoblast-like cells on laminin-coated polycarbonate membranes and equine collagen membranes under different culture conditions", [Online] 1 May 2010 (2010-05-01), JOURNAL OF BIOMEDICAL MATERIALS RESEARCH. PART A, WILEY PERIODICALS INC, HOBOKEN, NY, US, PAGE(S) 704 - 715, XP009159569, ISSN: 1549-3296 Retrieved from the Internet: URL:http://www3.interscience.wiley.com/cgi -bin/issn? DESCRIPTOR=PRINTISSN&VALUE=1549- 3296> [retrieved on 2009-07-16] * abstract ***

## Description

Cell culture models are used in vitro to simulate an equivalent biological in vivo situation of cell cultures, tissues or even whole organs in the pharmaceutical evaluation of biologically active substances or pharmaceuticals. Such in vitro models provide for the possibility to investigate and evaluate complex cell behaviour at defined experimental conditions, which allows to make conclusions about the situation in vivo. Biological systems that model and simulate in vitro-equivalent cell barriers for the investigation and/or evaluation of the growth of different interacting and/or interfering cell types is still another demanding task. Cell barriers occur in the human and animal body in order to separate different compartments such as the blood/brain barrier, the lung or mucous layers. Usually the compartments in the human or animal body have different milieus, such as, for example, blood/air, brain liquid/blood, intestinal lumen/blood.

By now, no appropriate biological in vitro system is known, which would be suitable for the investigation and/or evaluation of the native conditions as they occur at cell barriers in vivo. Most of the known in vitro systems do not sufficiently reflect the actual physiological conditions at particular cell barriers such as, for instance, the ratio of cell density between area and volume of the intestine. According to the literature, the length of the intestine, the diameter and its surface provide a ratio between cell-covered area and volume of approximately 17:1 (H.-J. Appell and C. Stang-Voss, Funktionelle Anatomie: Grundlagen sportlicher Leistung und Bewegung, 4th edn. (Springer, Berlin, 2008), pp. 150-152). In vitro systems that allow to simulate the situation in vivo should therefore provide a greater cell cultivation area and should at the same time occupy only a very small volume of medium. This allows for an improved assessment of molecules that are transported between the two compartments. Due to the higher cell surface ratio, their concentrations are significantly higher.

Many of the known in vitro systems are based on cell monocultures. In such systems, the cells grow to monolayers on the cultivation surface. The cells are morphologically and functionally polarized, i.e. no distinct apical or basolateral side exists. One of the most known in vitro systems for stimulating cell barriers is the so-called transwell system. Transwells consist of a device, comprising two chambers that are separated by a thin, porous membrane. Using the transwell system, the influence of the neuronal cell line PC 12 on the functionality of the intestinal epithel has been investigated (H. Satus, T. Yokoyama, N. Ogawa, Y. Fujiwara-Hatano, M. Shimizu, Biosci. Biotechnol. Biochem. 67 (6),1312 (2003). Although the transwell system can be used for a co-cultivation of two different cell types, it is not suitable to simulate a flow of cell culture medium at one or both sides of the cell barrier. In addition, the ratio between volume and cultivation surface is not equivalent to the physiological condition. Therefore, important biological parameters and physiological concentrations are not reflected in the transwell system.

A two chamber system which allows the investigation of cell behaviour under flow conditions has been developed (K. Christ, K. Williamson, K. Masters, K. Turner, Biomed Microdevices (2010), doi:10.1039/b813516a; Y. Imura, Y. Asano, K. Sato, E. Yoshimura, Analytical Sciences December 25, 1403 (2009)). However, the ratio between area/volume equals 1:1, and thus the system is not suitable to simulate the physiological behaviour of a mucous barrier. Furthermore, experiments under flow conditions are restricted to a few hours only until cell destruction or cell death occurs (J. Phelps, N. Depaola, Am J Physiol Hert Cric Physiol 278, 469 (2000); B. Wojciak-Stothard, A. Ridley, J. Cell Bio. 161, 429 (2003); Y. Shikata, A. Rios, K. Kawkitinarong, N. DePaola, J. Garcia, K. Birukov, Exp. Cell Res. (2004) doi: 10.1016/j.yexcr.2004.11.001). In some systems it is possible to cultivate cells (e.g. muscle cells) up to a maximum incubation time of two weeks (A. Torovskaia, X. Figueroa-Masot, A. Floch, Lab Chip (2005), doi:10.1039/b405719h). Furthermore, some micro fluidic in vitro systems have been developed to investigate the behaviour of cells that were opposed to stress under flow conditions, or to investigate their adherence (E. Anderson, M. Knothe Tate, BioMedical Engineering OnLine (2007), doi:10.1186/1475-925X-6-46; J. Green, T. Kniazeva, M. Abedi, D. Sokhey, M. Taslim, S. Murthy, Lab Chip (2009), doi:10.1039/b813516a; J. Haier, G. Nicolson, Clinical & Experimental Metastasis 17, 713 (1999). WO2009102751 discloses (claims 1, 4 and fig. 1) a microfluidic device as an vitro blood vessel model comprising two parallel channels separated by a porous membrane (which is a track-etched polycarbonate membrane) coated on one side by an endothelial cell layer and coated on the other side by a smooth muscle cell layer.

Although the use of known in vitro systems allows the analysis of certain aspects of cell interaction and their behaviour, no biological system so far has been described that could use a separate supply of different cell cultures with different media in order to simulate in vivo-equivalent cell barriers such as the blood/brain barrier, lung or mucous layers. In addition, the current systems do not reflect a physiological-equivalent ratio between volume and area required to simulate appropriate in vivo conditions. In particular, the intestinal barrier has a greater area than volume, which cannot be addressed by current systems. Furthermore, the current systems do not ensure a continuous flow on both sides of the barrier, i.e. they do not consider different physiological conditions in the separate compartments of the barrier.

It is thus the object of the present invention to provide a micro fluidic system for simulating in vivo-equivalent cell barriers which can be supplied with different media and/or biologically active substances or pharmaceuticals from different sides in order to implement different cells types in two separate compartments within the system. In addition, a constant media flow should be adjustable on both sides of the barrier and the ratio between volume and cell-covered area should be similar to the situation in vivo. Medium samples can be collected from both compartments and be further analysed in respect of relevant signalling molecules that are separated by each of the cell systems in dependence from the other, as well as molecules transported across the barrier.

The object of the invention is solved by a micro fluidic system according to claim 1 and/or a method for simulating in vivo-equivalent cell barriers according to claim 10.

The micro fluidic system for simulating in vivo-equivalent cell barriers according to the invention comprises a container with one or more micro fluidic modules. Each module consists of two micro-structured polycarbonate sheets (PC sheets) separated by a transmissible polycarbonate membrane (PC membrane), wherein both sides of the PC membrane are co-cultured with different cell types. The two PC sheets of a module contain continuous and separated micro channels that are arranged in parallel. The PC sheets with the separated micro channels are arranged orthogonally to each other, such that the channels cross each other. The two orthogonally arranged PC sheets are sealed in order to avoid cross-contamination. The PC sheets are preferably equipped with corresponding holes, which allow for an equal distribution of the individual media in all applied modules.

The orthogonal arrangement of the PC sheets induces a cross flow of media streams through the micro channels at the crossing sides of the micro channels in two different directions. Therefore, each micro channel-containing PC sheet can be equipped with different media for the delivery of substances to the both sides of the PC membrane, i.e. to the different cell cultures cultivated on the two membrane sides. At the crossing points, the cells can interact with each other, and thereby the transport of biologically active substances or pharmaceuticals through a cell barrier can be investigated and analysed. Preferably, the PC membrane is porous and allows cell-cell interactions between the cells cultivated on the both surfaces of the membrane.

The PC membrane preferably contains micro pores with pore sizes between 0,2 $\mu$m and 1,5 $\mu$m. In preferred embodiments, the pore sizes vary between 0,8 $\mu$m and 1,2 $\mu$m. Pore sizes of approximately 0,5 $\mu$m, 0,8 $\mu$m, 1,0 $\mu$m or 1,2 $\mu$m are preferred. Depending on the PC membrane, the pores are either symmetrically or asymmetrically orientated on the membrane surface. In order to induce the different fluid flows through the micro channels of the two PC sheets, the system is further equipped with a pump system, which allows for continuous apical and basolateral media supply of the growing cells by the micro channels of the PC sheets. Preferably, the circulation of media is achieved with a micro syringe pump and a tube system that feeds the micro channels with the respective medium.

The container according to the system of the invention may contain a varying number of modules, each module consisting of two orthogonally orientated PC sheets separated by a transmissible PC membrane. The PC sheets as utilized in the present invention can be produced by hot embossing using a form matrix allowing the generation of the micro channel structures. In a first embodiment, the PC sheets can be designed using UV lithography micro galvanoforming hot moulding. PC sheets produced by this method provide channel structures of 200 $\mu$m width, 50 $\mu$m height and a 40 mm length. Each micro channel has a volume of 0,4 $\mu$l and covers a surface of 8 mm$^2$. Each PC sheet produced by this method contains 120 channels. In this embodiment, the PC sheet size is 40 x 40 mm, the channel height is 50 $\mu$m, the channel width is 200 $\mu$m and the separation distance is 50 $\mu$m. A number of 75 channels per sheet can be accomplished. In a preferred embodiment, the supply inlets are preferably arranged at the side walls of the container. The modules are inserted within the container, and the edges are sealed to avoid cross contamination of the both fluid flows. In addition, the system is sealed from the exterior. In some embodiments, parameters that are different from the above mentioned and used in the described embodiments might be more suitable for individual applications.

Another way for producing PC sheets according to the invention is a method, which utilizes micro milling. Depending on the set-up and the milling cutter used, channel diameters down to 300 $\mu$m can be achieved. The distance between the micro channels can vary, depending on the number of channels integrated on the PC sheets, the diameters of each channel and their separation distances. In a preferred embodiment, the micro channels produced by micro milling have a width of 100 $\mu$m, a height of 50 $\mu$m and a length of 32 mm. Each PC sheet can contain 75 channels. The channels have a volume of 0,16 $\mu$l and cover a surface of 1,6 mm$^2$. For an upscaling of the production, mold injection technologies can be used.

In order to seal the PC sheets by means of surface pressure within the container, the micro channels of the PC sheets are sealed with sealing lips around the channel structure. By applying surface pressure on the PC sheets, the sealing lips of the sheets are pressed against the PC membrane and thereby sealed.

Depending on the module design, the fluid inlets at the container are arranged at suitable sides at the container such as at the side walls or on the cover of the container.

One advantage of the present invention is that different cell types can be cultured on the both sides of the PC membrane. Preferably, the cell cultures are selected from the group consisting of epithelial cells, endothelial cells, neuronal cells, heart cells, liver cells, lung cells, kidney cells, bladder cells, intestinal cells, blood cells, brain cells, immune cells, skin cells, fibroblasts.

In order to improve the adherence of the cells on the membrane surface, the PC membrane is preferably coated with one or more cell-adhesive substances, or a mixture thereof. Preferably, the cell adhesive substances are selected from the group consisting of Poly D lysine, Poly L lysine, collagen, Poly L ornithine, gelatin, laminin, fibronectin, tenascin, small peptides (RGD's) and other ECM proteins or antibodies directed against a cell surface epitope.

The ratio between the area covered by cells to the volume is at least ≥ 2:1, preferably ≥ 5:1, preferably ≥ 7:1, more preferably ≥ 10:1. Under appropriate cultivation conditions, the ratio between cell-covered area and volume of the cell cultures on both sides of the PC membrane is at least 10:1. Such a high ratio between cell-covered area and volume is in particular useful when resembling the in vivo situation of the human or animal intestine. This means that the system according to the present invention resembles the situation in vivo much better than current systems known in the art. The ratio achieved with the geometry according to this embodiment is 20:1.

In order to ensure a laminar flow of the micro fluidic system, the Reynold number (Re) should be < 2300. The flow condition can be described by the following formula:

$$\mathrm{Re} = \frac{d v \rho}{\eta} \qquad (1)$$

Re: Reynold number
d: channel dimension
$\eta$: viscosity of fluid (kg/m$^3$)
$v$: velocity of fluid (m/s)
p: density of fluid (kg/ms)

The dimension of the channel is defined by the hydraulic diameter:

$$dh = \frac{2bh}{b+h} \qquad (2)$$

dh: hydraulic diameter
$b$: channel width
h: channel height

Depending on the PC sheet design, the flow velocity may vary. Using the pc sheet designs according to the present embodiment, an optimal flow can be observed at flow rates between 1000 and 1500 μl/h. Other designs may require different flow rates.

In order to ensure appropriate cultivation conditions within the container of the micro fluidic system, it may be necessary to pre-incubate the container at cell culture conditions prior to inducing the flow of fluid through the micro channels. In order to induce a fluidic flow, the modules are connected with the pump system, which pumps the medium with a defined flow rate and velocity through the micro channels. Preferably, the flow rate per micro channel of the PC sheet is ranges between 4 and 5 μl/h. A pre-incubation period may be required in order to adjust and/or maintain the pH value of the medium within the biological system. The optimal flow rate is adjusted in accordance with the total number, diameter and distances of the micro channels. This adjustment allows for a continuous optimal media flow resulting in maximized cell vitality. If the flow rate is too high, cell destruction or cell death can be caused. Thus, the adjustment of the flow rate is crucial to ensure optimal cell culture conditions and cell vitality of the micro fluidic system of the invention.

The adherence time to allow the cells to adhere to the surface of the PC membrane must be sufficiently high to ensure vital cultivation of the cells on the membrane surface. In some situations, the adherence time is at least 4 hours. Preferably, the adherence time is between 4 and 24 hours, whereas 24 hours is most preferred. Adherence times up to 24 h do not interfere with the viability of the cells tested.

If the incubation time for adherence is too low, a significant number of cells may be washed out from the micro channels, which should be avoided. In addition, at short adherence times, the cultivation of cells may only occur at the crossing points of the PC membrane, but not throughout the structure of the micro channels. Furthermore, it has to be ensured that the cells adhere to the PC membrane surface rather than to the micro channel structure. The experiments as performed by the present inventors illustrate the adherence of cells on the both sides of the PC membrane, in particular when the PC membrane has been coated with cell-adhesive substances such as collagen or Poly L lysine.

In order to ensure transmissibility of biologically active substances or pharmaceuticals through the PC membrane, the membrane is equipped with pores of uniform or different sizes. Preferably, the pore size is adjusted in accordance with the cell barrier that is to be simulated. In a system that simulates the mucous layer of the intestine, the pore size of the PC membrane is preferably 1,2 μm. The surface of the PC membrane is preferably coated with collagen in order to enhance adherence of the cells to the membrane.

The number of modules contained in the container may vary. In most situations, the number of modules is at least two. Depending on the cell type, the cells are preferably pre-incubated in the system for at least 4 hours, preferably between 4 and 24 hours, whereas an incubation time of 24 hours is most preferred. The fluid flow is reduced following seeding of the cells onto the incubation surface. The flow should not be initiated too early in order to allow the cells to adhere on the membrane surface. By adapting the flow rate, vitality of the cells can be accurately adjusted. The best results are achieved at flow rates between 1200 μl/h and 1500 μl/h,

which corresponds to 4 and 5 $\mu$l per hour and channel. HT29 epithelial cells do not exhibit significant cell destruction when treated at a flow rate of 1200 $\mu$l/h for 5 days in the system.

It is also possible to establish a co-culture of different cell types on the both sides of the membrane. As shown in the experiment below, the reaction conditions can be adapted to establish a co-culture of HT29 cells and primary neuronal cells from the small intestine of newly bom rats. The primary neuronal cells are able to cultivate on the PC membrane in the vicinity of the micro channels and form a network within the channels upon inducing media flow and nutrient supply. Cell-cell interactions and the formation of nerve fibres can be observed. A more specific application can be based on the use of a three-dimensional gel. That means that the cells (preferably they were suspended in an extracellular matrix solution (collagen, laminin, ECM) and injected in one of the two compartments. There the solutions were allowed to gel and form a three-dimensional network, which is very close to the in vitro situation in the brain.

The system according to the present invention can be used for testing and evaluating the effect of biologically active substances or pharmaceuticals on cell growth and/or cell survival, in particular at cell barriers such as the blood-brain barrier, the lung or mucous layers. As the system resembles the in vivo situation of cell barriers within the human or animal body, the system can be suitably used for testing the effects of biologically active substances or pharmaceuticals at cell barriers, for instance on cell targeting, drug delivery or cell viability. The cell barrier of the system is accessible both from the apical and basolateral side, thereby a flow of different media types can be established in the two orthogonal flow directions of the both PC sheets. From each side of the PC sheets, an independent supply of medium can be ensured, simulating the situation in vivo, where the cell barriers separate the compartments in two different compartments such as blood/air, brain liquid/blood, intestinal lumen/blood. It is also possible to adjust a constant flow on one or both sides of the barrier. The system further provides for the possibility of a co-cultivation of different cell types on the both sides of the PC membrane. Cell-cell interactions can be observed via the transmissible pores of the PC membrane. The micro fluidic system according to the invention thus allows for the cultivation of different cell types because both sides of the cell barrier can be supplied with different media that are adjusted in accordance with the respective cell type and/or in vivo-equivalent situation. It is also of a great benefit of the system according to the invention that the adjustment of a continuous flow at both sides of the cell barrier is achievable. The system according to the invention can also be used for testing and evaluating the transport of substances across cell barriers.

Cells cultivated on the PC membrane grow, under flow conditions, to an intact cell culture resulting in the establishment of an in vivo-equivalent cell barrier. It is also of great benefit of the present invention that the system provides a ratio of the cell-covered area/volume of more than 10:1, which is, for instance, similar to the situation of the intestine in vivo.

It is a further advantage of the system according to the present invention that the cell cultures on the PC membrane can be exposed to a continuous flow over a period of several days. Thus, the micro fluidic system of the invention allows for a mono- and/or co-cultivation of cells under extended continuous media flow conditions. The cells of the cell cultures are in physical and physiological contact with each other via the porous PC membrane, which allows, for instance, the simulation of an invert mucous layer of the intestine.

The results as provided in the context of this invention demonstrate that the cells as used in the biological system require a balance between the supply with growth-stimulating substances and the maintenance of pressure under flow conditions in order to stay vital and to support their proliferation. If the flow is adjusted similar to the pressure of a capillary vessel as present in vivo, cell viability in the system was significantly enhanced.

In one preferred embodiment a plurality of individual micro fluid systems that represent individual biological barriers or tissues can be used. Preferably the micro fluid systems are serially interconnected in order to simulate the interdependence of various barriers or organ systems on each other.

The invention is further illustrated in the experiments and preferred embodiments described in the following.

## Material and Methods

## Production of micro-structured polycarbonate sheets (PC sheets)

The micro channels on the PC sheets were generated using a negative form matrix, which reflects the later channel structure. Two different negative matrices were produced using two different methods. The first method employs UV litography micro galvanoforming moulding. The form matrix design comprises a waver and a resist. For the production of the form matrix, the resist structure was covered with a metal, and the adhered waver was subsequently removed.

The second method uses micro milling of the PC sheets in which a milling cutter was used to introduce the micro channel structure in the PC sheets.

UV litography micro galvanoforming moulding results in a micro channel structure in which the channels have 200 $\mu$m width, 50 $\mu$m height and a length of 40 mm. Each channel has a volume of 0,4 $\mu$l and a surface of 8 mm$^2$. In total, 120 channels per sheet were moulded using this method. The sheet size is 40 x 40 mm, the channel height is 50 $\mu$m, the channel width is 200 $\mu$m, the separation distance between the channels is 50 $\mu$m. In total, 75 channels per sheet could be established.

Using the milling method, the distance between two parallel micro fluidic channels is 300 $\mu$m. The channels have a width of 100 $\mu$m, a height of 50 $\mu$m and a length of 32 mm. Each sheet contains 75 channels. The channels have a volume of 0,16 $\mu$l and a surface of 1,6 mm$^2$.

For sealing the PC sheets within the container, the channel structure was surrounded by a sealing lip. By applying surface pressure of the container, the respective sealing lips of the PC sheets are pressed against the membrane, thereby ensuring a tight sealing.

## Cell cultures

In order to establish a co-culture of different cell types, HT29 epithelial cells and primary neuronal cells from the intestine were utilized.

HT29 cells were incubated into a Dulbecco's modified Eagle's medium (DMEM Promocell), supplemented with 10 % fetal bovine serum (GBS, Sigma-Aldrich), 1 % non-essential amino acids (NEAA, Gibco) and 1 % penicillin streptomycin neomycin (PSN). The cells were incubated in 75 cm$^2$ bottles at a temperature of 37° C and an atmosphere of 5 % $CO_2$ in 95 % air. When grown to confluence, the cells were treated with 0,25 % trypsin-EDTA-solution (Gibco, Paisley, Scotland, UK) and used for the subsequent experiments.

The primary neuronal cells from the intestine of newly born rats were obtained by a modified protocol according to K-H. Schäfer and P. Mestres, Digestive Diseases and Sciences 45, 1631 (2000). The cells were cultivated in neurobasal A medium (Gibco) with 1 % PSN, 1 % bovine serum albumin (Sigma), 2 % B27 w RA, 0,1 % 2-mercaptoethanole (Gibco) and 0,25 % L-glutamine. For use in the micro fluidic system according to the invention, the neuronal cells were directly implemented in the cell container following preparation.

## Treatment of PC membranes

In order to increase adherence of the cells on the PC membrane surface, the cells were treated with different coatings. Poly-L-lysine (PLL 0,02 mg/ml A.dest; Sigma) and collagen R (Serva) were used at different concentrations. HT29 cells were sealed at a concentration of 50.000 cells per cm$^2$ and cultivated for two days. After this time, the sample surface covered with adhered cells was investigated and analysed.

## Cell cultivation within the micro fluidic system

HT29 cells and neuronal cells were used either in mono-culture or in co-culture. Cells were sealed at a density of 10.000 cells/cm$^2$. Four micro fluidic modules were inserted in the container. Flow rates were adjusted between 150 $\mu$l/h and 500 $\mu$l/min in order to optimize the growth conditions and enhance cell viability in the cell cultures. The pump and the supply means for applying the fluid flows were arranged outside of the incubator. The pH-value of the medium was analysed and, if necessary, adjusted. For analysis of cell viability, a life dead assay using calcein AM and propidium jodide was used. Since the PC membrane utilized in the present invention is not transparent, parts of the membrane covered with adhered cells thereto were used for the analysis using conventional SEM or TEM microscopy. For instance, morphology of the cells on the PC membrane was investigated using SEM microscopy. Cell behaviour in the membrane pores was investigated using TEM microscopy.

## Cell cultivation in the micro fluidic system

## Cell cultivation on PC membranes

In order to improve adherence of HT29 cells on the PC membranes, the surface of the PC membrane was treated with different cell adhesive substances. In the following experiments, the membranes were treated with Poly L lysine and collagen. The collagen coating was performed at a concentration of 0,2 mg/ml. The type of adhesive substance used for coating as well as its concentration may vary according to cell type. For HT29 cells, the use of collagen at a concentration of 0,2 mg/ml showed the most optimal results for growth of HT29 cells.

## Figure descriptions

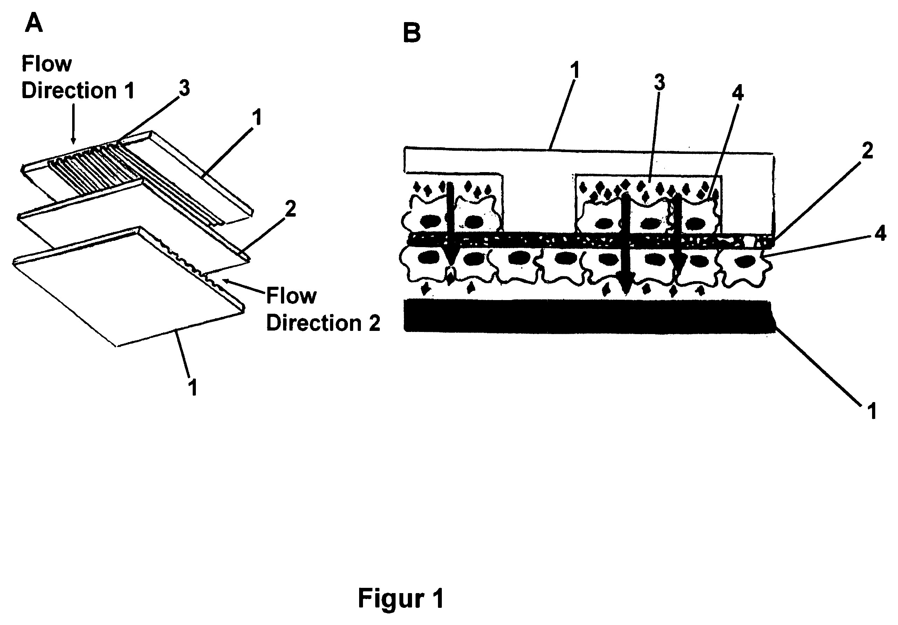

In Fig. 1, the composition of a micro fluidic module, consisting of two micro-structured polycarbonate sheets (PC sheets) (1) separated by a polycarbonate membrane (PC membrane) (2) is shown. The PC sheets(1) contain micro channels (3) that are orthogonally arranged and sealed against each other to induce a cross flow of different media streams through the micro channels (3) at their crossing sides (A): The two orthogonal flow directions are shown. At the crossing sides, a direct co-culture of cells (4) can be detected which is only separated by a transmissible PC membrane (2) with 17 $\mu$m strength (B): The PC membrane (2) contains micro pores which allow cell-cell interactions. The cells can interact at the crossing sides. The delivery of substances or pharmaceuticals from one side to the barrier to the other side can be investigated and analysed.

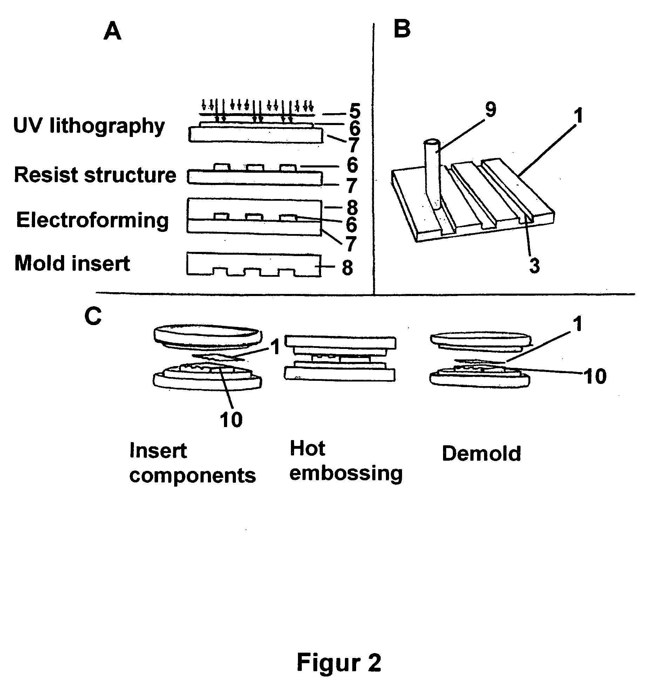

In Fig. 2, the production of a negative form matrix for the micro-structured PC sheets (1) is shown.

A: production of mold insert (10) using UV lithography micro galvanoforming moulding. The process steps using mask (5), resist (6) and wafer (7) is shown. The mold insert (10) is produced with metal (8) during electroforming.

B: production of PC sheets (1) using micro milling. The micro channel structure in the mold insert (10) is produced using an end milling cutter (9).

C: Hot embossing is used for the formation of the micro channel structures (3) in the PC sheets (1).

The mold insert (10) is used for the formation of the PC sheets (1).

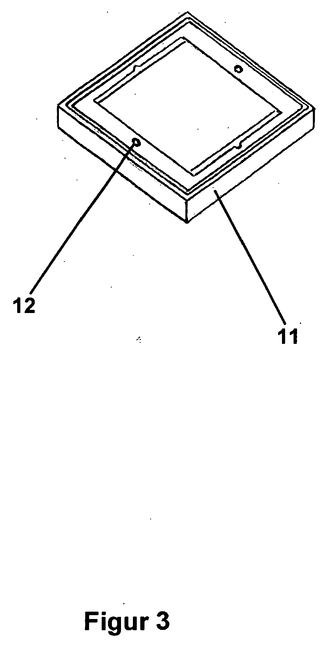

In Fig. 3, the mold (11) of the PC-sheet (1) used for the actual design is depicted (A). The individual PC sheets (1) can be supplied with media via an interconnecting hole (12) between the individual modules (B).

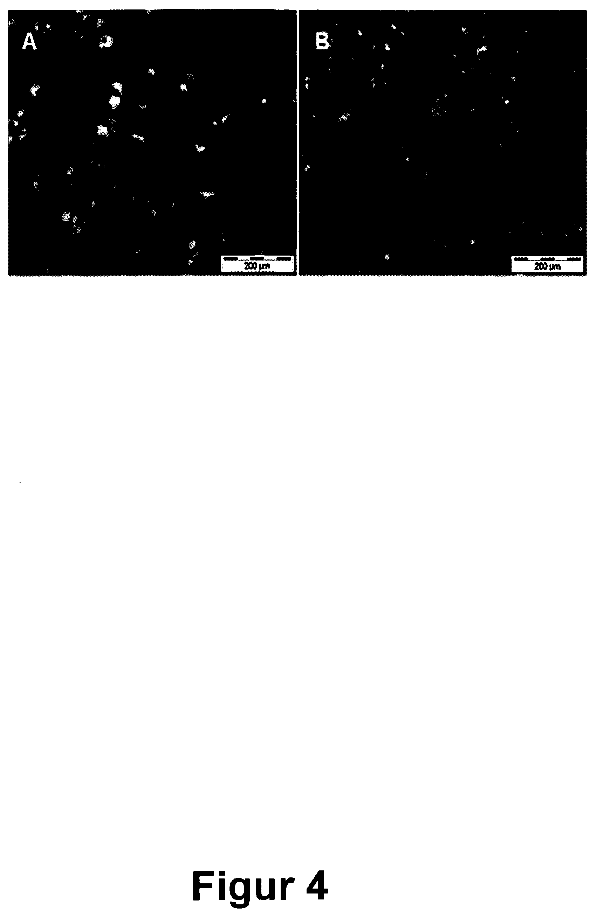

In Fig. 4, GFP-transfected HT29 cells were grown for 4 days in culture on a PC membrane (2) with pore sizes of 1,2 $\mu$m on (A) an untreated membrane surface and (B) on a membrane surface coated with collagen (0,2 mg/ml).

On the collagen-coated membrane, HT29 cells produced a confluent monolayer after 4 days of cultivation. On the untreated membrane, only 26 % of the membrane surface was coated with cells. Similar results were also achieved with primary neuronal cells. Depending on the cell barrier, both sides of the membrane can be coated either individually or uniformly. The kind of coating can be adjusted in accordance with the cell type used. Primary neuronal cells can be visualized on the PC membrane using anti-tubuline antibody staining. Depending on the cell type, coating of the PC membrane may be obligatory in order to avoid cell destruction or cell lost.

In order to adjust an optimal pH value of the medium, a pre-incubation of the cells at cell culture conditions was performed. Experiments using HT29 cells were performed in order to illustrate that the cells adhered to the membrane rather than to the channel surface data (not shown). HT29 cells were cultured on a collagen-coated PC membrane and marked with calcein AM and propidium jodide in order to determine their viability. The results clearly show that HT29 cells were viable and not destructed after 24 hours within the system. Before flow and medium support are established, a pre-incubation time of at least 4 hours took place in order to avoid flushing of cells from the system. Pre-incubation times of 24 hours were preferred. Flow rates between 51 $\mu$l/h to 60.000 $\mu$l/h in a system consisting of 4 micro fluidic modules were tested in order to determine the optimal flow rate for the cell culture. At a high flow rate greater than 30.000 $\mu$l/h, the cells were flushed away only after a short time. At flow rates less than 250 $\mu$l/h, the cells were heavily destructed after 48 hours in the system, similar to the situation when no flow was established. If the flow rate was greater than 500 $\mu$l/h, the number of vital cells increased significantly after 48 hours.

The number of vital cells could be increased by further increasing the flow rate. At a flow rate of 1000 $\mu$l/h, the cells could be cultivated for several days within the system. HT29 cells only showed a little number of destructed cells at a flow rate of 1000 $\mu$l/h upon 5 days of incubation. The best results were achieved at flow rates between 1200 $\mu$l/h and 1500 $\mu$l/h. No damages of cells could be detected if they were incubated for 5 days at a flow rate of 1200 $\mu$l/h.

The determined flow rates could be also adapted for other cell types such as primary neuronal cells.

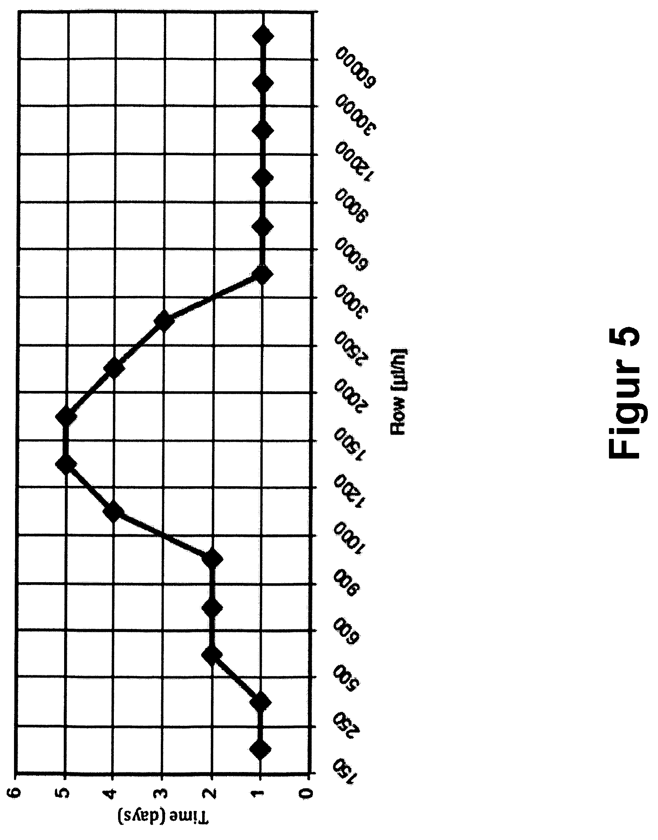

In Fig.5, the survival time in dependence of the flow rate is depicted. As shown by the diagram, flow rates between 1200 and 1500 $\mu$l/h result in optimal cell survival rates. The test was terminated after 5 days, and the cells growing under optimal flow rates where still completely alive.

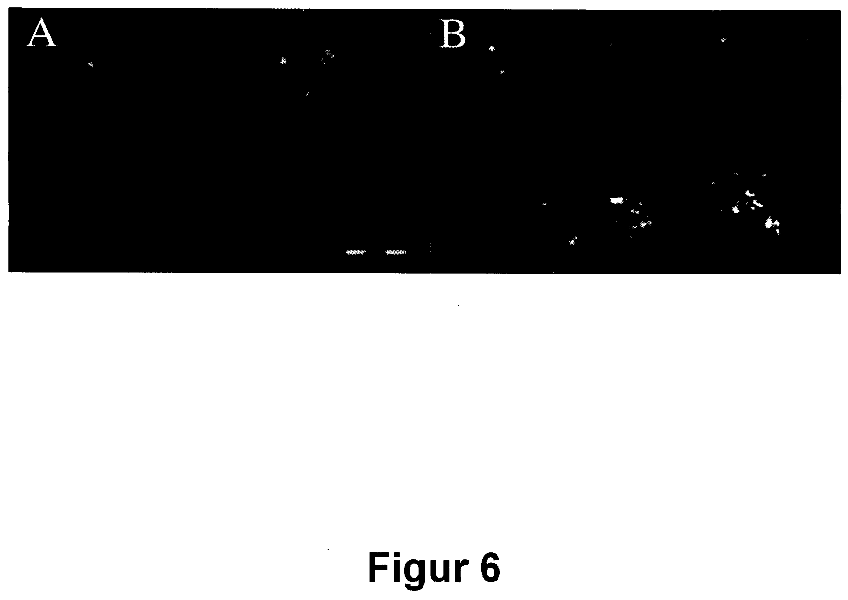

In Fig.6, the two cell types used in the actual application are depicted: Primary neuronal cells (A) and enterocytes HT-29 (B). The cells were cultivated for 48 hours. A total number of 4 modules was employed. The PC membrane had pore sizes of 1,2 $\mu$m pore diameter and were coated with collagen in the container. The flow was induced for 24 hours at 1200 $\mu$l/h after cell seeding.

## Claims

1. A micro fluidic system for simulating in vivo-equivalent cell barriers, comprising a container with one or more micro fluidic modules, wherein each module consists of two micro-structured polycarbonate sheets (PC sheets) (1) separated by a polycarbonate membrane (PC membrane) (2), wherein both sides of the PC membrane (2) are co-cultured with different cell types (4), **characterized in that** the two PC sheets (1) contain continuous and in parallel arranged micro channels (3), the PC sheets (1) with the micro channels (3) being arranged orthogonally and sealed against each other to induce a cross flow of different media streams through the micro channels (3) at the crossing sites of the micro channels (3).

2. The micro fluidic system according to claim 1, wherein the PC membrane (2) contains micro pores with pore sizes between 0,2 $\mu$m and 1,5 $\mu$m.

3. The micro fluidic system according to claim 1 or claim 2, wherein the system is further equipped with a pump system for continuous apical and basolateral media supply of the micro channels (3) of the PC sheets (1).

4. The micro fluidic system according to any one of the proceeding claims, wherein the cells of the cell cultures on the both sites of the PC membrane (2) are selected from the group consisting of epithelial cells, endothelial cells, neuronal cells, heart cells, liver cells, lung cells, kidney cells, bladder cells, intestinal cells, blood cells, brain cells, skin cells, immune cells, fibroblasts.

5. The micro fluidic system according to any one of the proceeding claims, wherein the micro channels (3) of the PC sheets (1) are sealed by means of sealing lips around the channel structure.

6. The micro fluidic system according to any one of the proceeding claims, wherein the PC membrane (2) is coated with one or more cell adhesive substances selected from the group consisting of Poly D lysine, Poly L lysine, collagen, Poly L ornithine, gelatin, laminin, fibronectin, tenascin, small peptides (RGD's) and other ECM proteins or antibodies directed against a cell surface epitope.

7. The micro fluidic system according to any one of the proceeding claims, wherein the ratio between cell-covered area and volume of the cell cultures (4) at the PC membrane (2) is at least 10:1.

8. The micro fluidic system according to any one of the proceeding claims, wherein the supplied media are cell growth media containing additional biologically active substances or pharmaceuticals.

9. The micro fluidic system according to any one of the proceeding claims, wherein the flow rate per micro channel of a PC sheet is not greater than 1000 μl/h.

10. A method for simulating in vivo-equivalent cell barriers, comprising the steps of:

- placing a at least one module consisting of a polycarbonate membrane (PC membrane) between two micro-structured polycarbonate sheets (PC sheets) within a container, wherein the two PC sheets contain continuous and in parallel arranged micro channels, the PC sheets with the micro channels being arranged orthogonally and sealed against each other,
- incubating of both sides of the PC membrane with different cell types for a time sufficient to allow the cells to adhere on the PC membrane surface,
- inducing a cross flow of different media streams through the micro channels of the PC sheets such that the cells adhered thereto are in contact with the media.

11. The method according to claim 10, wherein the both sides of the PC membrane are either uniformly or differently coated with one or more cell adhesive substances, and wherein the cells are allowed for a time and a temperature sufficient to adhere to both sides of the PC membrane.

12. The method according to any one of the proceeding claims, wherein one of the micro channels is filled with a gel-cell suspension resulting in the formation of a stable three-dimensional gel.

13. The method according to any one of the proceeding claims, wherein the flow rate is adjusted in accordance with the total number and diameter of the micro channels to allow a continuous optimal media flow at maximized cell vitality.

14. The method according to any one of the proceeding claims, wherein the container is pre-incubated at cell culture conditions prior to inducing the flow of fluid through the micro channels.

15. Use of a micro fluid system according to claim 1 or a method according to claim 10 for testing and evaluating the effect of biologically active substances or pharmaceuticals on cell growth and/or cell survival at cell barriers, and the transport of substances across cell barriers.

16. Use of a plurality of micro fluid systems according to claim 1 for stimulating the interdependence of various barriers or organ systems on each other, wherein the micro fluid systems are serially interconnected and represent individual biological barriers or tissues.

**Patentansprüche**

1. Mikrofluidsystem zur Stimulation von in vivo-äquivalenten Zellbarrieren, umfassend einen Behälter mit einem oder mehreren Mikrofluidmodulen, wobei jedes Modul aus zwei mikrostrukturierten Polycarbonatplatten (PC-Platten) (1) besteht, die von einer Polycarbonatmembran (PC-Membran) (2) beabstandet sind, wobei beide Seiten der PC-Membran (2) mit unterschiedlichen Zelltypen (4) co-kultiviert sind, **dadurch gekennzeichnet, dass** die zwei PC-Platten (1) kontinuierliche und parallel angeordnete Mikrokanäle (3) enthalten, wobei die PC-Platten (1) mit den Mikrokanälen (3) orthogonal angeordnet und zueinander abgedichtet sind, um einen Querstrom unterschiedlicher Medienströme durch die Mikrokanäle (3) an den Kreuzungsstellen der Mikrokanäle (3) zu ermöglichen.

2. Mikrofluidsystem nach Anspruch 1, wobei die PC-Membran (2) Mikroporen mit Porengrößen zwischen 0,2 μm und 1,5 μm enthält.

3. Mikrofluidsystem nach Anspruch 1 oder Anspruch 2, wobei das System ferner mit einem Pumpsystem versehen ist, um eine kontinuierlich apicale und basolaterale Medienversorgung der Mikrokanäle (3) der PC-Platten (1) zu ermöglichen.

4. Mikrofluidsystem nach einem der vorhergehenden Ansprüche, wobei die Zellen der Zellkulturen von beiden Seiten der PC-Membran (2) ausgewählt sind aus der Gruppe bestehend aus Epithelzellen, Endothelzellen, neuronalen Zellen, Herzzellen, Leberzellen, Lungenzellen, Nierenzellen, Blasenzellen, Darmzellen, Blutzellen, Gehirnzellen, Hautzellen, Immunzellen, Immunzellen, Fibroblasten.

**5.** Mikrofluidsystem nach einem der vorhergehenden Ansprüche, wobei die Mikrokanäle (3) der PC-Platten (1) mittels Dichtlippen um die Kanalstruktur abgedichtet sind.

**6.** Mikrofluidsystem nach einem der vorhergehenden Ansprüche, wobei die PC-Membran (2) mit einer oder mehreren zelladhäsiven Substanzen beschichtet sind, ausgewählt aus der Gruppe bestehend aus Poly-D-Lysin, Poly-L-Lysin, Kollagen, Poly-L-Ornithin, Gelatine, Laminin, Fibronectin, Tenascin, kleine Peptide (RGDs) und andere ECM-Proteine oder Antikörper, die gegen ein Zelloberflächenepitop gerichtet sind.

**7.** Mikrofluidsystem nach einem der vorhergehenden Ansprüche, wobei das Verhältnis zwischen der zellbedeckten Fläche und dem Volumen der Zellkulturen (4) auf der PC-Membran (2) wenigstens 10:1 beträgt.

**8.** Mikrofluidsystem nach einem der vorhergehenden Ansprüche, wobei die zur Verfügung gestellten Medien Zellwachstumsmedien sind, welche zusätzliche biologisch aktive Substanzen oder Pharmazeutika enthalten.

**9.** Mikrofluidsystem nach einem der vorhergehenden Ansprüche, wobei die Durchflussrate pro Mikrokanal einer PC-Platte nicht größer als 1000 μl/h ist.

**10.** Verfahren zur Stimulation von in vivo-äquivalenten Zellbarrieren, umfassend die Schritte:

- Anordnen wenigstens eines aus einer Polycarbonatmembran (PC-Membran) bestehenden Moduls zwischen zwei mikrostrukturierten Polycarbonatplatten (PC-Platten) innerhalb eines Behälters, wobei die beiden PC-Platten kontinuierliche und parallel zueinander angeordnete Mikrokanäle enthalten, wobei die PC-Platten mit den Mikrokanälen orthogonal angeordnet und zueinander abgedichtet sind,
- Inkubieren beider Seiten der PC-Membran mit unterschiedlichen Zelltypen für eine Zeitdauer die ausreicht, damit die Zellen auf der Oberfläche der PC-Membran anhaften,
- Induzieren eines Querstromes unterschiedlicher Medienströme durch die Mikrokanäle der PC-Platten derart, dass die daran angehafteten Zellen mit den Medien in Kontakt sind.

**11.** Verfahren nach Anspruch 10, wobei die beiden Seiten der PC-Membran entweder einheitlich oder unterschiedlich mit einer oder mehreren zelladhäsiven Substanzen beschichtet sind und wobei es den Zellen ermöglicht wird, dass sie für eine ausreichende Zeitdauer und Temperatur auf beiden Seiten der PC-Membran anhaften können.

**12.** Verfahren nach einem der vorhergehenden Ansprüche, wobei einer der Mikrokanäle mit einer Gel-Zell-Suspension gefüllt ist, was zur Bildung eines stabilen dreidimensionalen Gels führt.

**13.** Verfahren nach einem der vorhergehenden Ansprüche, wobei die Durchflussrate entsprechend der Gesamtanzahl und dem Durchmesser der Mikrokanäle angepasst wird, um einen kontinuierlichen optimalen Medienfluss bei maximierter Zellvitalität zu ermöglichen.

**14.** Verfahren nach einem der vorhergehenden Ansprüche, wobei der Behälter unter Zellkulturbedingungen vor dem Auslösen des Flüssigkeitsstroms durch die Mikrokanäle pre-inkubiert wird.

**15.** Verwendung eines Mikrofluidsystems nach Anspruch 1 oder einem Verfahren gemäß Anspruch 10 zum Testen und Evaluieren der Wirkung von biologisch aktiven Substanzen oder Pharmazeutika auf das Zellwachstum und/oder das Zellüberleben an Zellbarrieren, und dem Transport von Substanzen über Zellbarrieren.

**16.** Verwendung einer Vielzahl von Mikrofluidsystemen nach Anspruch 1 zur Stimulation der Wechselbeziehung von verschiedenen Barrieren oder Organsystemen zueinander, wobei die Mikrofluidsysteme miteinander in Reihe geschaltet sind und unterschiedliche biologische Barrieren oder Gewebe darstellen.

**Revendications**

**1.** Système microfluidique pour la simulation de barrières cellulaires équivalentes aux conditions *in vivo,* comprenant un récipient avec un ou plusieurs modules microfluidiques, où chaque module est constitué de deux feuilles de polycarbonate micro-structurées (feuilles PC) (1) séparées par une membrane de polycarbonate (membrane PC) (2), où les deux faces de la membrane PC (2) sont co-cultivées avec des types cellulaires différents (4), **caractérisé en ce que** les deux feuilles PC (1) contiennent des microcanaux disposés de façon continue et en parallèle (3), les feuilles PC (1) contenant les microcanaux (3) étant disposées orthogonalement et scellées l'une contre l'autre pour induire un débit croisé de flux de milieux différents à travers les microcanaux (3) aux sites de croisement des microcanaux (3).

**2.** Système microfluidique selon la revendication 1, où la membrane PC (2) contient des micropores de taille de pore comprise entre 0,2 μm et 1,5 μm.

**3.** Système microfluidique selon la revendication 1 ou la revendication 2, où le système est en outre équipé d'un système de pompe pour alimentation apicale et basolatérale en continu en milieu des microcanaux (3) des feuilles PC (1).

**4.** Système microfluidique selon l'une quelconque des revendications précédentes, où les cellules de culture cellulaire sur les deux sites de la membrane PC (2) sont choisies dans le groupe constitué par les suivantes : cellules épithéliales, cellules endothéliales, cellules neuronales, cellules cardiaques, cellules hépatiques, cellules pulmonaires, cellules rénales, cellules vésicales, cellules intestinales, cellules sanguines, cellules cérébrales, cellules cutanées, cellules immunitaires, fibroblastes.

**5.** Système microfluidique selon l'une quelconque des revendications précédentes, où les microcanaux (3) des feuilles PC (1) sont scellés par le biais de lèvres de scellage autour de la structure des canaux.

**6.** Système microfluidique selon l'une quelconque des revendications précédentes, où la membrane PC (2) est revêtue d'une ou de plusieurs substances d'adhésion cellulaire choisies dans le groupe constitué par les suivantes : Poly-D-lysine, Poly-L-lysine, collagène, Poly-L-ornithine, gélatine, laminine, fibronectine, ténascine, petits peptides (RGD) et autres protéines ECM ou anticorps dirigés contre un épitope de surface cellulaire.

**7.** Système microfluidique selon l'une quelconque des revendications précédentes, où le rapport entre la surface couverte par les cellules et le volume de culture cellulaire (4) au niveau de la membrane PC (2) est d'au moins 10:1.

**8.** Système microfluidique selon l'une quelconque des revendications précédentes, où les milieux fournis sont des milieux de croissance cellulaire contenant des substances biologiquement actives ou des produits pharmaceutiques supplémentaires.

**9.** Système microfluidique selon l'une quelconque des revendications précédentes, où le débit par microcanal d'une feuille PC ne dépasse pas 1000 μl/h.

**10.** Méthode de simulation de barrières cellulaires équivalentes aux conditions *in vivo,* comprenant les étapes suivantes :

- placement d'au moins un module constitué d'une membrane polycarbonate (membrane PC) entre deux feuilles de polycarbonate microstructurées (feuilles PC) à l'intérieur d'un récipient, où les deux feuilles PC contiennent des microcanaux disposés de façon continue et en parallèle, les feuilles PC avec les microcanaux étant disposés orthogonalement et scellées l'une contre l'autre,
- incubation des deux faces de la membrane PC avec des types de cellules différents pendant une durée suffisante pour permettre aux cellules d'adhérer à la surface de la membrane PC,
- induction d'un débit croisé de différents flux de milieux à travers les microcanaux des feuilles PC de sorte que les cellules collées à ces dernières soient en contact avec le milieu.

**11.** Méthode selon la revendication 10, où les deux faces de la membrane PC sont revêtues soit uniformément, soit différemment d'une ou de plusieurs substances d'adhésion cellulaire, et où les cellules sont laissées pendant une durée et à une température suffisantes pour adhérer aux deux faces de la membrane PC.

**12.** Méthode selon l'une quelconque des revendications précédentes, où l'un des microcanaux est rempli d'une suspension cellulaire en gel résultant en la formation d'un gel tridimensionnel stable.

**13.** Méthode selon l'une quelconque des revendications précédentes, où le débit est ajusté selon le nombre total et le diamètre des microcanaux pour obtenir un débit optimal continu avec une vitalité cellulaire maximisée.

**14.** Méthode selon l'une quelconque des revendications précédentes, où le récipient est pré-incubé à des conditions de culture cellulaire avant induction du débit fluidique à travers les microcanaux.

**15.** Utilisation d'un système microfluidique selon la revendication 1 ou méthode selon la revendication 10 pour tester et évaluer l'effet de substances biologiquement actives ou de produits pharmaceutiques sur la croissance cellulaire et/ou la survie cellulaire au niveau de barrières cellulaires, et le transport de substances à travers des barrières cellulaires.

**16.** Utilisation d'une multitude de systèmes microfluidiques selon la revendication 1 pour la stimulation de l'interdépendance de diverses barrières ou de divers systèmes d'organes les uns avec les autres, où les systèmes microfluidiques sont interconnectés en série et représentent des barrières biologiques ou des tissus individuels.

**A**

Flow
Direction 1

3

1

2

Flow
Direction 2

1

1

**B**

1

3

4

2

4

1

Figur 1

EP 2 628 790 B1

**A**

UV lithography

Resist structure

Electroforming

Mold insert

5
6
7

6
7

8
6
7

8

**B**

9

1

3

**C**

1

10

Insert
components

Hot
embossing

Demold

1

10

**Figur 2**

**Figur 3**

# Figur 4

**Figur 5**

EP 2 628 790 B1

Figur 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009102751 A

### Non-patent literature cited in the description

- **H.-J. APPELL ; C. STANG-VOSS.** Funktionelle Anatomie: Grundlagen sportlicher Leistung und Bewegung. Springer, 2008, 150-152
- **H. SATUS ; T. YOKOYAMA ; N. OGAWA ; Y. FUJIWARA-HATANO ; M. SHIMIZU.** *Biosci. Biotechnol. Biochem.,* 2003, vol. 67 (6), 1312
- **K. CHRIST ; K. WILLIAMSON ; K. MASTERS ; K. TURNER.** *Biomed Microdevices,* 2010
- **Y. IMURA ; Y. ASANO ; K. SATO ; E. YOSHIMURA.** *Analytical Sciences,* 25 December 2009, 1403
- **J. PHELPS ; N. DEPAOLA.** *Am J Physiol Hert Cric Physiol,* 2000, vol. 278, 469
- **B. WOJCIAK-STOTHARD ; A. RIDLEY.** *J. Cell Bio.,* 2003, vol. 161, 429
- **Y. SHIKATA ; A. RIOS ; K. KAWKITINARONG ; N. DEPAOLA ; J. GARCIA ; K. BIRUKOV.** *Exp. Cell Res.,* 2004
- **A. TOROVSKAIA ; X. FIGUEROA-MASOT ; A. FLOCH.** *Lab Chip,* 2005
- **E. ANDERSON ; M. KNOTHE TATE.** *BioMedical Engineering OnLine,* 2007
- **J. GREEN ; T. KNIAZEVA ; M. ABEDI ; D. SOKHEY ; M. TASLIM ; S. MURTHY.** *Lab Chip,* 2009
- **J. HAIER ; G. NICOLSON.** *Clinical & Experimental Metastasis,* 1999, vol. 17, 713
- **K-H. SCHÄFER ; P. MESTRES.** *Digestive Diseases and Sciences,* 2000, vol. 45, 1631